(19) Europäisches Patentamt
European Patent Office
Office européen des brevets

(11) EP 0 878 461 B1

(12) # EUROPÄISCHE PATENTSCHRIFT

(45) Veröffentlichungstag und Bekanntmachung des Hinweises auf die Patenterteilung:
**10.04.2002 Patentblatt 2002/15**

(51) Int Cl.[7]: **C07C 209/18**

(21) Anmeldenummer: **98107389.3**

(22) Anmeldetag: **23.04.1998**

(54) **Verfahren zur Herstellung von 3,5-Difluoranilin**

Process for the preparation of 3,5-difluoroaniline

Procédé pour la préparation of 3,5-difluoroaniline

(84) Benannte Vertragsstaaten:
**CH FR GB LI**

(30) Priorität: **15.05.1997 DE 19720341**

(43) Veröffentlichungstag der Anmeldung:
**18.11.1998 Patentblatt 1998/47**

(73) Patentinhaber: **Clariant GmbH**
**65929 Frankfurt am Main (DE)**

(72) Erfinder:
- **Pfirmann, Ralf, Dr.**
  **64347 Griesheim (DE)**
- **Krause, Stefan, Dr.**
  **65929 Frankfurt (DE)**

(56) Entgegenhaltungen:
**EP-A- 0 562 435**

- **PATENT ABSTRACTS OF JAPAN vol. 018, no. 019 (C-1152), 13. Januar 1994 (1994-01-13) & JP 05 255206 A (TORAY IND INC), 5. Oktober 1993 (1993-10-05)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 878 461 B1

Printed by Jouve, 75001 PARIS (FR)

## Beschreibung

Die vorliegende Erfindung betrifft ein vorteilhaftes Verfahren zur Herstellung von 3,5-Difluoranilin.

3,5-Difluoranilin stellt eine wichtige Verbindung unter anderem zur Herstellung von Industriechemikalien dar.

Aufgrund der Bedeutung von 3,5-Difluoranilin als Synthesebaustein - wie beispielsweise aus J. Amer. Chem. Soc. 81, 94-101 (1959) hervorgeht - hat es nicht an Versuchen gefehlt, Synthesen zur Herstellung von 3,5-Difluoranilin zu entwickeln.

So ist aus WO 96/02493, ausgehend von 2,4-Difluoranilin, eine über fünf Stufen verlaufende Synthese bekannt. Man setzt zunächst 2,4-Difluoranilin mit Acetanhydrid um, nitriert das resultierende Acetanilid mittels $HNO_3$/$H_2SO_4$, spaltet den Acetylrest ab und erhält 2,4-Difluor-6-nitro-anilin. Durch Umsetzung des 2,4-Difluor-6-nitroanilins mit Natriumnitrit läßt sich die Aminogruppe entfernen und man erhält 3,5-Difluornitrobenzol, das sich durch Reduktion in das 3,5-Difluoranilin überführen läßt.

Eine über vier Stufen verlaufende Synthese ist der EP 562 435 zu entnehmen. Man setzt zunächst 2,4,5-Trichlornitrobenzol mit einem Alkalimetallfluorid zu 5-Chlor-2,4-difluornitrobenzol um. Durch chlorierende Denitrierung läßt sich das 5-Chlor-2,4-difluornitrobenzol in das 1,3-Dichlor-4,6-difluorbenzol umwandeln. Das 1,3-Dichlor-4,6-difluorbenzol wird zu 2,6-Dichlor-3,5-difluornitrobenzol nitriert. Aus dem 2,6-Dichlor-3,5-difluomitrobenzol läßt sich durch Umsetzung mit Wasserstoff unter Chlorwasserstoffabspaltung und Reduktion der Nitrogruppe das gewünschte 3,5-Difluoranilin herstellen.

In der EP 460 639 ist eine dreistufige Herstellung von 3,5-Difluoranilin beschrieben. Man geht von 5-Chlor-2,4,6-trifluorisophthalsäure aus, decarboxyliert diese zu 2-Chlor-1,3,5-trifluorbenzol. Durch Umsetzung des 2-Chlor-1,3,5-trifluorbenzols mit Kupfer und Wasser bei 300°C läßt sich das 1,3,5-Trifluorbenzol herstellen. Gemäß Beispiel 6 der EP 460 639 setzt man 1,3,5-Trifluorbenzol mit Ammoniak gesättigtem Methanol bei 200°C über 60 Stunden um und erhält das 3,5-Difluoranilin.

Die vorstehend beschriebenen Herstellungsverfahren verlaufen über mehrere Stufen und erfordern einen dementsprechend hohen Aufwand. Die hierfür benötigten Ausgangsstoffe - 2,4-Difluoranilin, 2,4,5-Trichlornitrobenzol und 5-Chlor-2,4,6-trifluorisophthalsäure - stellen Verbindungen dar, die nicht leicht zugänglich sind, sondern zumeist mit Hilfe über mehrere Stufen verlaufender Synthesen erhältlich sind. Dadurch erhöht sich der Arbeitsaufwand zur Herstellung von 3,5-Difluoranilin zusätzlich.

In Hinblick hierauf besteht ein Interesse, ein Verfahren zur Herstellung von 3,5-Difluoranilin bereitzustellen, das diese Nachteile nicht aufweist. Es soll einen einfachen, kurzen Syntheseweg eröffnen, sich mit einem vertretbaren Arbeitsaufwand ausführen lassen und das gewünschte 3,5-Difluoranilin in akzeptablen Ausbeuten liefern.

Diese Aufgabe wird gelöst durch ein Verfahren zur Herstellung von 3,5-Difluoranilin. Es ist dadurch gekennzeichnet, daß man 3,5-Difluorchlorbenzol mit Ammoniak in Anwesenheit eines Lösungsmittels in Gegenwart einer Kupferverbindung und wenigstens eines Metalles ausgewählt aus der Gruppe Kupfer, Eisen, Kobalt, Nickel, Chrom, Molybdän und Zink, bei 100 bis 250°C umsetzt.

Das für das erfindungsgemäße Verfahren benötigte 3,5-Difluorchlorbenzol läßt sich durch Umsetzung von 1,3,5-Trichlorbenzol mit einem Alkalimetallfluorid in Gegenwart eines Lösungsmittels oder eines geeigneten Katalysator herstellen.

Die Umsetzung von 1,3,5-Trichlorbenzol mit KF respektive einem KF/CsF-Gemisch in Dimethylsulfoxid führt zu 3,5-Difluorchlorbenzol. Diese Art der Umsetzung erfordert allerdings sehr hohe Temperaturen, nämlich 280 respektive 275°C, und liefert das 3,5-Difluorchlorbenzol mit 47,7 % respektive 40,5 % Ausbeute (Vergleiche auch Shiley, Dickerson and Finger, J. Fluorine Chem., 2 (1972/73) Seiten 19 bis 26). Derart hohe Reaktionstemperaturen stellen allerdings an die Verschleißfestigkeit der verwendeten Reaktorbehälter und Rührwerke hohe Ansprüche und können die Bildung von Nebenprodukten begünstigen.

Eine besonders günstige Variante dieser Umsetzung ist in der nicht vorveröffentlichten deutschen Patentanmeldung (Aktenzeichen 196 31 854.8) beschrieben. Diese Anmeldung betrifft ein Verfahren zur Herstellung von Fluor enthaltenden Verbindungen, indem man eine Verbindung, die gegen Fluor austauschbares Halogen enthält, mit einem Fluorid oder einem Gemisch von Fluoriden der allgemeinen Formel

$$MeF \qquad (I),$$

worin Me für ein Erdalkalimetallion, $NH_4^+$-ion oder Alkalimetallion, in Anwesenheit einer Verbindung oder eines Gemisches von Verbindungen der allgemeinen Formel

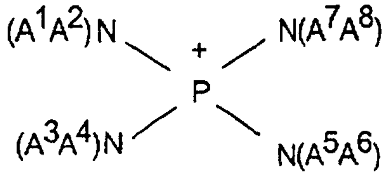

$B^-$ (II)

worin $A^1$, $A^2$, $A^3$, $A^4$, $A^5$, $A^6$, $A^7$, $A^8$ unabhängig voneinander, gleich oder verschieden sind und für ein geradkettiges oder verzweigtes Alkyl oder Alkenyl mit 1 bis 12 Kohlenstoffatomen, Cycloalkyl mit 4 bis 8 Kohlenstoffatomen, für ein Aryl mit 6 bis 12 Kohlenstoffatomen, ein Aralkyl mit 7 bis 12 Kohlenstoffatornen stehen, oder $A^1$ $A^2$, $A^3A^4$, $A^5A^6$, $A^7A^8$ unabhängig voneinander, gleich oder verschieden sind und direkt oder über O oder N-$A^9$ miteinander zu einem Ring mit 3 bis 7 Ringgliedern verbunden sind, $A^9$ für ein Alkyl mit 1 bis 4 Kohlenstoffatomen steht und $B^-$ einen einwertigen Säurerest oder das Äquivalent eines mehrwertigen Säurersts bedeutet in Anwesenheit oder Abwesenheit eines Lösungsmittels bei einer Temperatur von 40 bis 260°C umsetzt.

Die vorstehend genannte Verbindung der Formel (II) respektive das Gemisch derartiger Verbindungen erweist sich als geeigneter Katalysator für die Umsetzung beispielsweise von 1,3,5-Trichlorbenzol mittels Alkalimetallfluoriden zu 3,5-Difluorchlorbenzol.

Man setzt als Fluorid der Formel (I) Kalziumfluorid, Ammoniumfluorid, Lithiumfluorid, Natriumfluorid, Kaliumfluorid, Rubidiumfluorid, Cäsiumfluorid oder ein Gemisch derselben, insbesondere Lithiumfluorid, Natriumfluorid, Kaliumfluorid, Rubidiumfluorid, Cäsiumfluorid oder ein Gemisch derselben, bevorzugt Natriumfluorid, Kaliumfluorid, Cäsiumfluorid oder ein Gemisch derselben, besonders bevorzugt Kaliumfluorid, Cäsiumfluorid oder ein Gemisch derselben ein. Häufig genügt es, Kaliumfluorid allein einzusetzen.

Die Verbindungen der Formel (II) lassen sich beispielsweise durch Umsetzung von Phosphorpentachlorid mit Dialkylaminen herstellen. Aus der nachfolgenden Gleichung ist die Umsetzung unter Verwendung von Dimethylamin zu entnehmen.

$$PCl_5 + HN(CH_3)_2 \rightarrow P[N(CH_3)_2]_4\ Cl$$

Man kann aber auch Phosphorpentachlorid stufenweise mit unterschiedlichen sekundären Aminen, beispielsweise Dialkylaminen, zur Reaktion bringen, um unsymmetrisch substituierte Verbindungen der Formel (II) zu erhalten. Weitere Möglichkeiten, Verbindungen der Formel (II) zu synthetisieren, sind von R. Schwesinger et al., Angew. Chem. 103 (1991) 1376 und R. Schwesinger et al., Chem. Ber. 127 (1994) 2435 bis 2454 beschrieben.

Üblicherweise setzt man eine Verbindung der Formel (II), worin $B^-$ für $F^-$, $Cl^-$, $Br^-$, $J^-$, $HF_2^-$, $BF_4^-$, $C_6H_5SO_3^-$, p-$CH_3$-$C_6H_5SO_3^-$, $HSO_4^-$, $PF_6^-$, $CF_3SO_3^-$, insbesondere für F-, $Cl^-$, Br, $J^-$, $HF_2^-$, $BF_4^-$, steht, ein.

Man setzt die Verbindung der Formel (II) in einer Menge von 0,5 bis 35, insbesondere 1 bis 30, bevorzugt 3 bis 25 Gew.-%, bezogen auf die Verbindung die gegen Fluor austauschbares Halogen enthält, ein.

Um nicht ausschließlich auf die vorstehenden Angaben in Gew.-% angewiesen zu sein, kann man in einer Vielzahl von Fällen, die Verbindung der Formel (II) in einer Menge von 0,1 bis 3, insbesondere von 0,4 bis 5, bevorzugt 0,5 bis 1 Mol-%, bezogen auf die Verbindung, die gegen Fluor austauschbares Halogen enthält, einsetzen. Diese Mengen erweisen sich üblicherweise als ausreichend.

Ohne Anspruch auf Vollständigkeit zu erheben, seien als Beispiele für Verbindungen der Formel (II) genannt.

Tetrakis(dimethylamino)phosphoniumchlorid
Tetrakis(diethylamino)phosphoniumchlorid
Tetrakis(dimethylamino)phosphoniumbromid
Tetrakis(diethylamino)phosphoniumbromid
Tetrakis(dipropylamino)phosphoniumchlorid oder -bromid
Tris(diethylamino)(dimethylamino)phosphoniumchlorid oder -bromid
Tetrakis(dibutylamino)phosphoniumchlorid oder -bromid
Tris(dimethylamino)(diethylamino)phosphoniumchlorid oder -bromid
Tris(dimethylamino)(cyclopentylamino)phosphoniumchlorid oder -bromid
Tris(dimethylamino)(dipropylamino)phosphoniumchlorid oder - bromid
Tris(dimethylamino)(dibutylamino)phosphoniumchlorid oder -bromid
Tris(dimethylamino)(cyclohexylamino)phosphoniumchlorid oder -bromid

Tris(dimethylamino)(diallylamino)phosphoniumchlorid oder -bromid
Tris(dimethylamino)(dihexylamino)phosphoniumchlorid oder -bromid
Tris(diethylamino)(dihexylamino)phosphoniumchlorid oder -bromid
Tris(dimethylamino)(diheptylamino)phosphoniumchlorid oder -bromid
Tris(diethylamino)(diheptylamino)phosphonium chlorid oder -bromid
Tetrakis(pyrrolidino)phosphoniumchlorid oder -bromid
Tetrakis(piperidino)phosphoniumchlorid oder -bromid
Tetrakis(morpholino)phosphoniumchlorid oder -bromid
Tris(piperidino)(diallylamino)phosphoniumchlorid oder -bromid
Tris(pyrrolidino)(ethylmethylamino)phosphoniumchlorid oder -bromid
Tris(pyrrolidino)(diethylamino)phosphoniumchlorid oder -bromid.

Man kann als Katalysator eine Verbindung der Formel (II) oder ein Gemisch zweier oder mehrerer Verbindungen der Formel (II) verwenden. Besonders einfach gestaltet sich dies, wenn man Gemische von Verbindungen der Formel (II), wie sie bei der Synthese anfallen, verwendet.

Das Verfahren läßt sich wie zuvor bereits erwähnt, in Anwesenheit oder Abwesenheit eines Lösungsmittels durchführen. Werden Lösungsmittel verwendet, so sind sowohl dipolar aprotische und aprotische als auch protische Lösungsmittel geeignet. Geeignete dipolar aprotische Lösungsmittel sind beispielsweise Dimethylsulfoxid (DMSO), Dimethylsulfon, Sulfolan (TMS), Dimethylformamid (DMF), Dimethylacetamid, 1,3-Dimethylimidazolin-2-on, N-Methylpyrrolidon, Hexamethylphosphorsäuretriamid, Acetonitril und Benzonitril. Diese Lösungsmittel können auch als Gemisch Anwendung finden.

Geeignete aprotische Lösungsmittel ohne ausgeprägten dipolaren Charakter sind aromatische Kohlenwasserstoffe oder chlorierte aromatische Kohlenwasserstoffe, beispielsweise Benzol, Toluol, ortho-, meta-, para-Xylol, technische Gemische isomerer Xylole, Ethylbenzol, Mesitylen, ortho-, meta-, para-Chlortoluol, Chlorbenzol und ortho-, meta-, para-Dichlorbenzol. Es können auch Gemische dieser Lösungsmittel verwendet werden.

Das aprotische oder dipolar aprotische Lösungsmittel kann in beliebigen Mengen, beispielsweise 5 bis 500 Gew.-%, verwendet werden, bevorzugt werden allerdings geringe Mengen im Bereich von 5 bis 30 Gew.-%, bezogen auf die Verbindung, die gegen Fluor austauschbares Halogen enthält. Bei der Verwendung von protischen Lösungsmitteln liegen die eingesetzten Mengen im Bereich von 0,1 bis 5, bevorzugt 0,1 bis 2 Gew.-%, bezogen auf die Verbindung, die gegen Fluor austauschbares Halogen enthält.

Die Reaktionstemperatur hängt auch von der Art der Verbindung, die gegen Fluor austauschbares Halogen enthält, ab. So erfordern vergleichsweise reaktionsträge Verbindungen in der Regel höhere Reaktionstemperaturen, während vergleichsweise reaktive Ausgangsstoffe sich bereits bei relativ niedrigen Temperaturen mit Erfolg umsetzen lassen.

Die Umsetzung von 1,3,5-Trichlorbenzol kann man bereits bei Temperaturen von beispielsweise 170 bis 210°C mit gutem Erfolg durchführen. Vergleiche auch das Beispiel im experimentellen Teil.

Mit Hilfe der vorstehend genannten Katalysatoren (Verbindungen der Formel (II)) gelingt es also, das vergleichsweise recht reaktionsträge 1,3,5-Trichlorbenzol mit einem Alkalimetallfluorid zu dem gewünschten 3,5-Difluorchlorbenzol umzusetzen. Die vorangegangenen Angaben bezüglich der Umsetzung von 1,3,5-Trichlorbenzol dienen dazu, das Verfahren der nicht vorveröffentlichten deutschen Anmeldung (Aktenzeichen 196 31 854.8) näher zu erläutern.

Durch das erfindungsgemäße Verfahren eröffnet sich nunmehr ein kurzer direkter Syntheseweg, der, ausgehend von 1,3,5-Trichlorbenzol, in lediglich zwei Schritten zum gewünschten 3,5-Difluoranilin führt. Man stellt zunächst aus 1,3,5-Trichlorbenzol 3,5-Difluorchlorbenzol her und setzt anschließend das 3,5-Difluorchlorbenzol entsprechend der vorliegenden Erfindung zu 3,5-Difluoranilin um. Dieser Syntheseweg ist kurz und, da er von 1,3,5-Trichlorbenzol, das in technischen Mengen verfügbar ist, ausgeht, von besonderem Interesse.

Die Umsetzung verläuft entsprechend dem nachfolgenden Reaktionsschema:

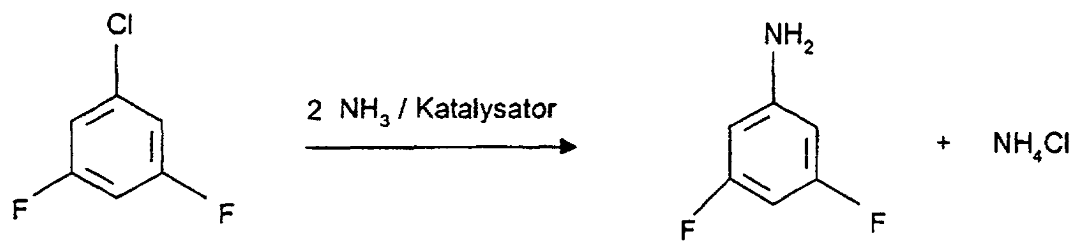


3,5-Difluorchlorbenzol 3,5-Difluoranilin

Mittels des erfindungsgemäßen Verfahrens gelingt es überraschenderweise , den Chlorsubstituenten des 3,5-Difluorchlorbenzols mit hoher Selektivität gegen eine Aminogruppe auszutauschen. Dies war nicht zu erwarten, da das 3,5-Difluorchlorbenzol nicht weniger als drei Halogensubstituenten enthält, nämlich zwei Fluorreste und einen Chlorrest, die jeweils gegen eine Aminogruppe ausgetauscht werden können. Vielmehr mußte damit gerechnet werden, daß sich auch Produkte bilden, in denen ein Fluorsubstituent oder zwei Halogensubstituenten, beispielsweise der Chlorrest und ein Fluorrest jeweils gegen eine Aminogruppe ausgetauscht worden sind.

Es war nicht zu erwarten, daß es mit Hilfe des erfindungsgemäßen Verfahrens gelingen würde, ausschließlich den Chlorsubstituenten in 3,5-Difluorchlorbenzol mit hoher Selektivität durch eine Aminogruppe zu ersetzen.

Man kann Ammoniak und 3,5-Difluorchlorbenzol in stöchiometrischem Verhältnis oder mit einem sehr großen Ammoniaküberschuß umsetzen. Üblicherweise setzt man Ammoniak und 3,5-Difluorchlorbenzol im Molverhältnis (1 bis 200):1, insbesondere (1:100):1 ein.

In einer Vielzahl von Fällen hat es sich als günstig erwiesen, Ammoniak und 3,5-Difluorchlorbenzol im Molverhältnis (5 bis 30):1, insbesondere (10 bis 20):1 einzusetzen.

Man kann Ammoniak in gasförmiger oder flüssiger Form oder gelöst in einem Lösungsmittel einsetzen.

Man führt die Umsetzung in Anwesenheit eines Lösungsmittels durch. Als Lösungsmittel setzt man Wasser oder ein Wasser und ein wasserlösliches Lösungsmittel enthaltendes Gemisch, insbesondere Wasser ein.

Als wasserlösliches Lösungsmittel eignen sich protische Lösungsmittel wie Alkohole, insbesondere aliphatische Alkohole mit 1 bis 4 Kohlenstoffatomen, und/oder aprotische Lösungsmittel wie Tetrahydrofuran, Dioxan, Sulfolan, N-Methylpyrrolidon, Dimethylacetamid, Dimethylformamid, Diethylacetamid, Diethylformamid, N,N,N,N-Tetramethylharnstoff.

Verwendet man ein Wasser und ein wasserlösliches Lösungsmittel enthaltendes Gemisch, so sollte dieses Gemisch wenigstens 60, insbesondere wenigstens 80 Gew.-% Wasser, üblicherweise 60 bis 99, insbesondere 75 bis 98 Gew.-% Wasser enthalten. Dadurch wird sichergestellt, daß Ammoniak in ausreichendem Maße gelöst wird, um die Umsetzung erfolgreich ablaufen zu lassen.

Es ist besonders einfach, Ammoniak in Form einer wäßrigen Lösung zu verwenden.

Man setzt Ammoniak in Form einer 10 bis 35, insbesondere 15 bis 30 Gew.-% Ammoniak enthaltenden wäßrigen Lösung ein.

Das für die Umsetzung erforderliche Katalysatorsystem besteht aus einer Kupferverbindung und wenigstens einem Metall, insbesondere einer Kupferverbindung und einem Metall, ausgewählt aus der Gruppe Kupfer, Eisen, Kobalt, Nickel, Chrom, Molybdän und/oder Zink.

Man setzt als Kupferverbindung eine Kupfer(I)verbindung, eine Kupfer(II)verbindung oder ein Gemisch derselben, insbesondere eine Kupfer(I)verbindung oder ein Gemisch derselben ein.

Man setzt als Kupferverbindung ein Kupfer(I)salz oder Kupfer(I)oxid, insbesondere Kupfer(I)chlorid, Kupfer (I)bromid oder Kupfer(I)jodid, bevorzugt Kupfer(I)chlorid oder Kupfer(I)jodid ein.
Besonders geeignet ist Kupfer(I)chlorid.

Man führt die Umsetzung in Gegenwart wenigstens eines Melalles ausgewählt aus der Gruppe Kupfer, Eisen, Kobalt und Nickel, insbesondere ausgewählt aus der Gruppe Kupfer, Eisen und Kobalt durch.

Man kann als Metall vorzugsweise Kupfer oder Eisen einsetzen.
Es hat sich als besonders günstig erwiesen, in einer Reihe von Fällen Kupfer als Metall einzusetzen.

An dieser Stelle sei ausdrücklich erwähnt, daß das Katalysatorsystem neben der Kupferverbindung ein Metall der vorstehend genannten Gruppe enthält. Nur diese Kombination aus Kupferverbindung und Metall hat sich als selektiv wirkendes Katalysatorsystem erwiesen, das das gewünschte 3,5-Difluoranilin in guten Ausbeuten zugänglich macht.

Üblicherweise setzt man die Kupferverbindung und 3,5-Difluorchlorbenzol im Molverhältnis (0,05 bis 1,5):1, insbesondere (0,1 bis 0,6):1, bevorzugt (0,15 bis 0,4):1 ein.

Man setzt in der Regel (0,05 bis 1,5), insbesondere (0,1 bis 0,6), bevorzugt (0,15 bis 0,4) g-Atom Metall je Mol 3,5-Difluorchlorbenzol ein.

Es hat sich häufig als ausreichend erwiesen, die Umsetzung bei 130 bis 200, insbesondere 140 bis 190°C durchzuführen.

Das Verfahren läßt sich kontinuierlich oder diskontinuierlich durchführen. Es kann unter Normaldruck oder erhöhtem Druck ausgeübt werden. Besonders einfach ist es, das Verfahren unter dem sich jeweils unter den angewendeten Reaktionsbedingungen einstellendem Eigendruck ablaufen zu lassen. Hierbei sind Drücke von 5 bis 100, insbesondere 10 bis 60 bar üblich.

Als Ausgangsmaterial verwendet man vorteilhafterweise ein aus 1,3,5-Trichlorbenzol durch Fluor-Chloraustausch hergestelltes 3,5-Difluorchlorbenzol, beispielsweise ein entsprechend der vorstehend genannten deutschen Anmeldung (Aktenzeichen 196 31 854.8) hergestelltes 3,5-Difluorchlorbenzol.

Die nachfolgenden Beispiele beschreiben die Erfindung näher, ohne sie zu beschränken.

Experimenteller Teil

Herstellung von 3,5-Difluorchlorbenzol

(Beispiel 12 der nicht vorveröffentlichten deutschen Anmeldung (Aktenzeichen 196 31 854.8))

Herstellung von 1-Fluor-3,5-dichlorbenzol und 1,3-Difluor-5-chlorbenzol (3,5-Difluorchlorbenzol) durch Umsetzung von 1,3,5-Trichlorbenzol mittels Tetrakis(diethylamino)-phosphoniumbromid

Man legt in einem 500 ml Vierhalskolben, der mit Thermometer, Ankerrührer und Rückflußkühler mit Blasenzähler bestückt ist, 181,5 g (1 mol) 1,3,5-Trichlorbenzol, 136,8 g (2,4 mol) Kaliumfluorid und 7,98 g (0,02 mol) Tetrakis (diethylamino)-phosphoniumbromid vor. Anschließend erhitzt man unter Rühren auf die vorgegebene Reaktionstemperatur von 180°C und läßt 10 Stunden reagieren. Nach Beendigung der Umsetzung läßt man abkühlen, löst das Reaktionsgemisch in Methylenchlorid, filtriert unlösliche Bestandteile (Salze wie KCl, KF) ab und reinigt die Wertprodukte (1-Fluor-3,5-dichlorbenzol und 1,3-Difluor-5-chlorbenzol (3,5-Difluorchlorbenzol)) durch fraktionierte Destillation.

Der Umsatz beträgt 100 %. 1-Fluor-3,5-dichlorbenzol: 50 % Ausbeute; 1,3-Difluor-5-chlorbenzol (3,5-Difluorchlorbenzol): 37 % Ausbeute.

Beispiel 1 bis 6

Herstellung von 3,5-Difluoranilin durch Umsetzung von 3,5-Difluorchlorbenzol in Gegenwart einer Kupferverbindung und eines Metalles

In einem 100 ml Autoklaven mit Tefloneinsatz werden die in der nachfolgenden Tabelle angegebenen Mengen 3,5-Difluorchlorbenzol, Kupferverbindung (Beispiel 1 bis 3: CuCl); Beispiele 4 und 6: CuJ; Beispiel 5: $Cu_2O$), Metall (Beispiele 1 bis 5: Kupferpulver; Beispiel 6: Stahlspäne) und wäßrige Ammoniaklösung (25 Gew.-% Ammoniak) vorgelegt. Der Autoklav wird geschlossen und die Umsetzung wird unter Rühren mit der in der Tabelle angegebenen Temperatur und Zeit durchgeführt. Der Inhalt des Autoklaven wird abgekühlt und über eine Glasfritte filtriert. Das Filtrat wird mit etwa 150 ml Methylenchlorid extrahiert. Anschließend wird der in der Tabelle ausgewiesene Gehalt 3,5-Difluoranilin und 3,5-Difluorchlorbenzol gaschromatographisch mit innerem Standard ermittelt.

Falls das Wertprodukt (3,5-Difluoranilin) isoliert werden soll, destilliert man den Methylenchloridextrakt. Hierbei geht zunächst bei Normaldruck Methylenchlorid als Vorlauf über, anschließend wird bei etwa 116°C und Normaldruck eventuell noch vorhandenes 3,5-Difluorchlorbenzol und anschließend bei etwa 150°C und 20 mbar 3,5-Difluoranilin abdestilliert.

Vergleichsbeispiel A

Herstellung von 3,5-Difluoranilin durch Umsetzung von 3,5-Difluorchlorbenzol in Gegenwart einer Kupferverbindung, jedoch ohne Zusatz eines Metalles

In einem 100 ml Autoklav mit Tefloneinsatz wird die in der nachfolgenden Tabelle angegebene Menge 3,5-Difluorchlorbenzol, CuJ als Kupferverbindung und wäßrige Ammoniaklösung (25 Gew.-% Ammoniak) vorgelegt und es wird, wie in Beispiel 1 bis 6 beschrieben, gearbeitet. Auf den Zusatz eines Metalles wird verzichtet.

Es bilden sich in hohem Maße teerartige Produkte. Der Umsatz ist fast quantitativ, der Gehalt an 3,5-Difluoranilin beträgt lediglich 50 % der Theorie.

Vergleichsbeispiel B

Herstellung von 3,5-Difluoranilin durch Umsetzung von 3,5-Difluorchlorbenzol in Gegenwart von Kupfer als Metall, jedoch ohne Zusatz einer Kupferverbindung

In einem 100 ml Autoklav mit Tefloneinsatz wird die in der nachfolgenden Tabelle angegebene Menge 3,5-Difluorchlorbenzol, Kupferpulver als Metall und wäßrige Ammoniaklösung (25 Gew.-% Ammoniak) vorgelegt und es wird, wie in Beispiel 1 bis 6 beschrieben, gearbeitet. Auf den Zusatz einer Kupferverbindung wird verzichtet. Neben Zersetzungsprodukten (etwa 19 %) bilden sich erhebliche Mengen Fluoranilin (etwa 20 %) und 3-Fluor-5-chloranilin (etwa 30 %). Die vorstehend genannten, in Klammer gesetzten Prozentangaben beziehen sich auf eine Abschätzung nichtgeeichter GC-Analysedaten. Der Umsatz beträgt 90 % der Theorie, der Gehalt an 3,5-Difluoranilin beträgt lediglich 21 % der Theorie.

Die bei den Beispielen 1 bis 6 und bei den Vergleichsbeispiels A und B angewendeten Reaktionsbedingungen (Einsatzmengen, Temperaturen, Zeiten) und Analysendaten (Gehalt 3,5-Difluoranilin und 3,5-Difluorchlorbenzol auf Basis GC-Analyse mit innerem Standard) sind in der nachfolgenden Tabelle zusammengestellt.

Tabelle

| Beispiele | Einsatzmengen | | | | Reaktionsbedingungen | | Gehalt Reaktionsprodukt nach GC * | |
|---|---|---|---|---|---|---|---|---|
| | 3,5-Difluorchlorbenzol | Kupferverbindung | Metall | Ammoniak als 25 %ige Lsg. | Temp. | Zeit | Gehalt 3,5-Difluoranilin | Gehalt 3,5-Difluorchlorbenzol (Edukt) |
| 1 | 0,05 mol | 0,015 mol CuCl | 0,015 mol Cu-Pulver | 0,50 mol | 150°C | 24 h | 78 % d.Th. | 9 % d.Th. |
| 2 | 0,05 mol | 0,015 mol CuCl | 0,015 mol Cu-Pulver | 0,50 mol | 200°C | 12 h | 74 % d.Th. | 1 % d.Th. |
| 3 | 0,05 mol | 0,010 mol CuCl | 0,010 mol Cu-Pulver | 0,75 mol | 175°C | 18 h | 76 % d.Th. | 2 % d.Th. |
| 4 | 0,05 mol | 0,010 mol CuJ | 0,010 mol Cu-Pulver | 0,75 mol | 180°C | 24 h | 76 % d.Th. | 1 % d.Th. |
| A | 0,05 mol | 0,010 mol CuJ | -- | 0,75 mol | 175°C | 18 h | 50 % d.Th. | 1 % d.Th. ** |
| B | 0,05 mol | -- | 0,010 mol Cu-Pulver | 0,75 mol | 175°C | 18 h | 21 % d.Th. | 10 % d.Th. *** |
| 5 | 0,05 mol | 0,005 mol $Cu_2O$ | 0,010 mol Cu-Pulver | 0,75 mol | 180°C | 24 h | 45 % d.Th. | 30 % d.Th. |
| 6 | 0,05 mol | 0,010 mol CuJ | 1 g Stahlspäne | 0,75 mol | 180°C | 24 h | 75 % d.Th. | 4 % d.Th. |

* gaschromatographische Analyse mit innerem Standard

** Beim Einsatz von CuJ ohne Zusatz von Metall tritt in starkem Maße unselektive Zersetzung auf

*** Das Hauptprodukt bei der Umsetzung nur mit Kupferpulver ist 3-Fluor-5-chloranilin.

d.Th. = der Theorie

**Patentansprüche**

1. Verfahren zur Herstellung von 3,5-Difluoranilin, **dadurch gekennzeichnet, daß** man 3,5-Difluorchlorbenzol mit Ammoniak in Anwesenheit eines Lösungsmittels in Gegenwart einer Kupferverbindung und wenigstens eines Metalles, ausgewählt aus der Gruppe Kupfer, Eisen, Kobalt, Nickel, Chrom, Molybdän und Zink, bei 100 bis 250°C umsetzt.

2. Verfahren nach Anspruch 1, **dadurch gekennzeichnet, daß** man Ammoniak und 3,5-Difluorchlorbenzol im Molverhältnis (1 bis 100):1 einsetzt.

3. Verfahren nach Anspruch 1 oder 2, **dadurch gekennzeichnet, daß** man Ammoniak und 3,5-Difluorchlorbenzol im Molverhältnis (5 bis 30):1 einsetzt.

4. Verfahren nach einem oder mehreren der Ansprüche 1 bis 3, **dadurch gekennzeichnet, daß** man Ammoniak und 3,5-Difluorchlorbenzol im Molverhältnis (10 bis 20):1 einsetzt.

5. Verfahren nach einem oder mehreren der Ansprüche 1 bis 4, **dadurch gekennzeichnet, daß** man als Lösungsmittel Wasser oder ein Wasser und ein wasserlösliches Lösungsmittel enthaltendes Gemisch einsetzt.

6. Verfahren nach einem oder mehreren der Ansprüche 1 bis 5, **dadurch gekennzeichnet, daß** man als Lösungsmittel Wasser einsetzt.

7. Verfahren nach einem oder mehreren der Ansprüche 1 bis 6, **dadurch gekennzeichnet, daß** man Ammoniak in Form einer 10 bis 35 Gew.-% Ammoniak enthaltenden wäßrigen Lösung einsetzt.

8. Verfahren nach einem oder mehreren der Ansprüche 1 bis 7, **dadurch gekennzeichnet, daß** man Ammoniak in Form einer 15 bis 30 Gew.-% Ammoniak enthaltenden wäßrigen Lösung einsetzt.

9. Verfahren nach einem oder mehreren der Ansprüche 1 bis 8, **dadurch gekennzeichnet, daß** man als Kupferverbindung eine Kupfer(I)verbindung, eine Kupfer(II)verbindung oder ein Gemisch derselben einsetzt.

10. Verfahren nach einem oder mehreren der Ansprüche 1 bis 9, **dadurch gekennzeichnet, daß** man als Kupferverbindung ein Kupfer(I)salz oder Kupfer(I)oxid einsetzt.

11. Verfahren nach einem oder mehreren der Ansprüche 1 bis 10, **dadurch gekennzeichnet, daß** man als Kupferverbindung Kupfer(I)chlorid, Kupfer(I)bromid oder Kupfer(I)jodid einsetzt.

12. Verfahren nach einem oder mehreren der Ansprüche 1 bis 11, **dadurch gekennzeichnet, daß** man die Umsetzung in Gegenwart wenigstens eines Metalles ausgewählt aus der Gruppe Kupfer, Eisen, Kobalt und Nickel durchführt.

13. Verfahren nach einem oder mehreren der Ansprüche 1 bis 12, **dadurch gekennzeichnet, daß** man die Umsetzung in Gegenwart wenigstens eines Metalles ausgewählt aus der Gruppe Kupfer, Eisen und Kobalt durchführt.

14. Verfahren nach einem oder mehreren der Ansprüche 1 bis 13, **dadurch gekennzeichnet, daß** man als Metall Kupfer oder Eisen einsetzt.

15. Verfahren nach einem oder mehreren der Ansprüche 1 bis 14, **dadurch gekennzeichnet, daß** man als Metall Kupfer einsetzt.

16. Verfahren nach einem oder mehreren der Ansprüche 1 bis 15, **dadurch gekennzeichnet, daß** man die Kupferverbindung und 3,5-Difluorchlorbenzol im Molverhältnis (0,05 bis 1,5):1 einsetzt.

17. Verfahren nach einem oder mehreren der Ansprüche 1 bis 16, **dadurch gekennzeichnet, daß** man die Kupferverbindung und 3,5-Difluorchlorbenzol im Molverhältnis (0,1 bis 0,6):1, insbesondere (0,15 bis 0,4):1 einsetzt.

18. Verfahren nach einem oder mehreren der Ansprüche 1 bis 17, **dadurch gekennzeichnet, daß** man (0,05 bis 1,5) g-Atom Metall je Mol 3,5-Difluorchlorbenzol einsetzt.

**19.** Verfahren nach einem oder mehreren der Ansprüche 1 bis 18, **dadurch gekennzeichnet, daß** man (0,1 bis 0,6), insbesondere (0,15 bis 0,4) g-Atom Metall je Mol 3,5-Difluorchlorbenzol einsetzt.

**20.** Verfahren nach einem oder mehreren der Ansprüche 1 bis 19, **dadurch gekennzeichnet, daß** man die Umsetzung bei 130 bis 200°C durchführt.

**21.** Verfahren nach einem oder mehreren der Ansprüche 1 bis 20, **dadurch gekennzeichnet, daß** man die Umsetzung bei 140 bis 190°C durchführt.

**22.** Verfahren nach einem oder mehreren der Ansprüche 1 bis 21, **dadurch gekennzeichnet, daß** man ein aus 1,3,5-Trichlorbenzol durch Fluor-Chlor-Austausch hergestelltes 3,5-Difluorchlorbenzol einsetzt.

**Claims**

**1.** A process for the preparation of 3,5-difluoroaniline, which comprises reacting 3,5-difluorochlorobenzene with ammonia in the presence of a solvent in the presence of a copper compound and at least one metal selected from the group consisting of copper, iron, cobalt, nickel, chromium, molybdenum and zinc, at 100 to 250°C.

**2.** The process as claimed in claim 1, wherein ammonia and 3,5-difluorochlorobenzene are employed in the molar ratio (1 to 100):1.

**3.** The process as claimed in claim 1 or 2, wherein ammonia and 3,5-difluorochlorobenzene are employed in the molar ratio (5 to 30):1.

**4.** The process as claimed in one or more of claims 1 to 3, wherein ammonia and 3,5-difluorochlorobenzene are employed in the molar ratio (10 to 20):1.

**5.** The process as claimed in one or more of claims 1 to 4, wherein the solvent employed is water or a mixture containing water and a water-soluble solvent.

**6.** The process as claimed in one or more of claims 1 to 5, wherein the solvent employed is water.

**7.** The process as claimed in one or more of claims 1 to 6, wherein ammonia is employed in the form of a 10 to 35% by weight ammonia-containing aqueous solution.

**8.** The process as claimed in one or more of claims 1 to 7, wherein ammonia is employed in the form of a 15 to 30% by weight ammonia-containing aqueous solution.

**9.** The process as claimed in one or more of claims 1 to 8, wherein the copper compound employed is a copper(I) compound, a copper(II) compound or a mixture thereof.

**10.** The process as claimed in one or more of claims 1 to 9, wherein the copper compound employed is a copper(I) salt or copper(I) oxide.

**11.** The process as claimed in one or more of claims 1 to 10, wherein the copper compound employed is copper (I) chloride, copper (I) bromide or copper (I) iodide.

**12.** The process as claimed in one or more of claims 1 to 11, wherein the reaction is carried out in the presence of at least one metal selected from the group consisting of copper, iron, cobalt and nickel.

**13.** The process as claimed in one or more of claims 1 to 12, wherein the reaction is carried out in the presence of at least one metal selected from the group consisting of copper, iron and cobalt.

**14.** The process as claimed in one or more of claims 1 to 13, wherein the metal employed is copper or iron.

**15.** The process as claimed in one or more of claims 1 to 14, wherein the metal employed is copper.

**16.** The process as claimed in one or more of claims 1 to 15, wherein the copper compound and 3,5-difluorochlorobenzene are employed in the molar ratio (0.05 to 1.5):1.

**17.** The process as claimed in one or more of claims 1 to 16, wherein the copper compound and 3,5-difluorochlorobenzene are employed in the molar ratio (0.1 to 0.6):1, in particular (0.15 to 0.4):1.

**18.** The process as claimed in one or more of claims 1 to 17, wherein (0.05 to 1.5) g atom of metal are employed per mole of 3,5-difluorochlorobenzene.

**19.** The process as claimed in one or more of claims 1 to 18, wherein (0.1 to 0.6), in particular (0.15 to 0.4) g atom of metal are employed per mole of 3,5-difluorochlorobenzene.

**20.** The process as claimed in one or more of claims 1 to 19, wherein the reaction is carried out at 130 to 200°C.

**21.** The process as claimed in one or more of claims 1 to 20, wherein the reaction is carried out at 140 to 190°C.

**22.** The process as claimed in one or more of claims 1 to 21, wherein a 3,5-difluorochlorobenzene prepared from 1,3,5-trichlorobenzene by fluorine-chlorine exchange is employed.

## Revendications

**1.** Procédé pour la préparation de 3,5-difluoroaniline, **caractérisé en ce qu'**on met à réagir le 3,5-difluorochlorobenzène avec de l'ammoniac en présence d'un solvant en présence d'un composé du cuivre et d'au moins un métal, choisi dans le groupe du cuivre, du fer, du cobalt, du nickel, du chrome, du molybdène et du zinc, entre 100 et 250°C.

**2.** Procédé selon la revendication 1, **caractérisé en ce qu'**on utilise l'ammoniac et le 3,5-difluorochlorobenzène dans le rapport molaire de (1 à 100) : 1.

**3.** Procédé selon la revendication 1 ou 2, **caractérisé en ce qu'**on utilise l'ammoniac et le 3,5-difluorochlorobenzène dans le rapport molaire de (5 à 30) : 1.

**4.** Procédé selon une ou plusieurs des revendications 1 à 3, **caractérisé en ce qu'**on utilise l'ammoniac et le 3,5-difluorochlorobenzène dans le rapport molaire de (10 à 20) : 1.

**5.** Procédé selon une ou plusieurs des revendications 1 à 4, **caractérisé en ce qu'**on utilise comme solvant l'eau ou l'eau et un mélange contenant des solvants hydrosolubles.

**6.** Procédé selon une ou plusieurs des revendications 1 à 5, **caractérisé en ce qu'**on utilise l'eau comme solvant.

**7.** Procédé selon une ou plusieurs des revendications 1 à 6, **caractérisé en ce qu'**on utilise l'ammoniac sous la forme d'une solution aqueuse contenant de 10 à 35 % en poids d'ammoniac.

**8.** Procédé selon une ou plusieurs des revendications 1 à 7, **caractérisé en ce qu'**on utilise l'ammoniac sous la forme d'une solution aqueuse contenant de 15 à 30 % en poids d'ammoniac.

**9.** Procédé selon une ou plusieurs des revendications 1 à 8, **caractérisé en ce qu'**on utilise comme composé du cuivre un composé du cuivre (I), un composé du cuivre (II) ou un mélange de ceux-ci.

**10.** Procédé selon une ou plusieurs des revendications 1 à 9, **caractérisé en ce qu'**on utilise comme composé du cuivre un sel de cuivre (I) ou un oxyde de cuivre (II).

**11.** Procédé selon une ou plusieurs des revendications 1 à 10, **caractérisé en ce qu'**on utilise comme composé du cuivre le chlorure de cuivre (I), le bromure de cuivre (I) ou l'iodure de cuivre (I).

**12.** Procédé selon une ou plusieurs des revendications 1 à 11, **caractérisé en ce qu'**on réalise la réaction en présence d'au moins un métal choisi parmi le groupe du cuivre, du fer, du cobalt et du nickel.

13. Procédé selon une ou plusieurs des revendications 1 à 12, **caractérisé en ce qu'**on réalise la réaction en présence d'au moins un métal choisi parmi le groupe du cuivre, du fer et du cobalt.

14. Procédé selon une ou plusieurs des revendications 1 à 13, **caractérisé en ce qu'**on utilise comme métal le cuivre ou le fer.

15. Procédé selon une ou plusieurs des revendications 1 à 14, **caractérisé en ce qu'**on utilise le cuivre comme métal.

16. Procédé selon une ou plusieurs des revendications 1 à 15, **caractérisé en ce qu'**on utilise le composé de cuivre et le 3,5-difluorochlorobenzène dans le rapport molaire de (0,05 à 1,5) : 1.

17. Procédé selon une ou plusieurs des revendications 1 à 16, **caractérisé en ce qu'**on utilise le composé de cuivre et le 3,5-difluorochlorobenzène dans le rapport molaire de (0,1 à 0,6) : 1, en particulier de (0,15 à 0,4) : 1.

18. Procédé selon une ou plusieurs des revendications 1 à 17, **caractérisé en ce qu'**on utilise (0,05 à 1,5) g d'atome métal par mole de 3,5-difluorochlorobenzène.

19. Procédé selon une ou plusieurs des revendications 1 à 18 **caractérisé en ce qu'**on utilise (0,1 à 0,6), en particulier (0,15 à 0,4) g d'atome métal par mole de 3,5-difluorochlorobenzène.

20. Procédé selon une ou plusieurs des revendications 1 à 19, **caractérisé en ce qu'**on réalise la réaction entre 130 et 200°C.

21. Procédé selon une ou plusieurs des revendications 1 à 20, **caractérisé en ce qu'**on réalise la réaction entre 140 et 190°C.

22. Procédé selon une ou plusieurs des revendications 1 à 21, **caractérisé en ce qu'**on utilise le 3,5-difluorochlorobenzène préparé à partir de 1,3,5-trichlorobenzène par échange fluor-chlore.